Europäisches Patentamt

European Patent Office

Office européen des brevets

(11) Publication number: **0 123 291**
**A2**

## (12) EUROPEAN PATENT APPLICATION

(21) Application number: **84104447.2**

(22) Date of filing: **19.04.84**

(51) Int. Cl.³: **A 61 K 45/02**

(30) Priority: 20.04.83 JP 69850/83
09.09.83 JP 166380/83

(43) Date of publication of application:
31.10.84 Bulletin 84/44

(84) Designated Contracting States:
DE FR GB IT

(71) Applicant: KYOWA HAKKO KOGYO CO., LTD.
6-1, Ohte-Machi 1-chome
Chiyoda-ku Tokyo-to(JP)

(72) Inventor: Kato, Yasuki
1188, Shimotogari Nagaizumi-cho
Sunto-gun Shizuoka-ken(JP)

(72) Inventor: Ohshima, Rie
525-4, Shimotogari Nagaizumi-cho
Sunto-gun Shizuoka-ken(JP)

(72) Inventor: Tanaka, Naoko
1020-1, Nishikumando
Numazu-shi Shizuoka-ken(JP)

(72) Inventor: Hayakawa, Eiji
495-15, Chabatake
Susono-shi Shizuoka-ken(JP)

(72) Inventor: Moriyama, Masuo
2658-9, Togo, Shimokanuki
Numazu-shi Shizuoka-ken(JP)

(72) Inventor: Kondo, Akira
1937-26, Hayashinoshita Shimokanuki
Numazu-shi Shizuoka-ken(JP)

(74) Representative: Casalonga, Axel et al,
BUREAU D.A. CASALONGA OFFICE JOSSE & PETIT
Baaderstrasse 12-14
D-8000 München 5(DE)

(54) Method for stabilizing interferon.

(57) A method for stabilizing interferon which involves the addition of an alkali metal salt or magnesium chloride to interferon as a stabilizer. The stability of the interferon may be further enhanced by the addition of serum albumin and by freeze-drying.

Also, a method for stabilizing interferon which comprises bringing interferon into contact with a saccharide that is selected from the group consisting of dextran, chondroitin sulfuric acid, starch, glycogen, inulin, dextrin and alginic acid salt.

Specification


The present invention relates to a method for stabilizing interferon and to compositions which contain interferon and a saccharide or inorganic salt for stabilizing the interferon.

Interferon is a physiologically active substance useful as a therapeutic agent because of its biological activities such as antivirus, anti-cancer and like activities. Interferon is classified into type α, type β, type γ, etc., according to the physiological and physicochemical properties, and the origin.

Interferon has been produced by incubating cells of animals, in particular, cells of humans, but since the supply of such cells was limited, there has been developed a process for producing interferon which comprises cloning an interferon gene by recombinant DNA technology, introducing it into a microorganism; e.g., _Escherichia_ _coli_, and incubating the microorganism.

Interferon is a protein or a sugar protein which is unstable and its biological activity is decreased during clinical application. Heretofore, it is known that pH adjustment, addition of serum albumin, addition of soluble lanthanide or calcium salts (U.S. Patent 4,252,791), addition of polyethylenic nonionic surfactants, antibiotics, carboxylic acid, chelating agents, amino acids, etc. (Japanese Published Unexamined Patent Application No. 102519/1980), addition of polyethylenic nonionic surfactants, antibiotics, chelating agents, amino acids, etc., addition of acid sugars and addition of sulfur-containing reducing agents (European Patent Publication No. 80879), addition of HSA (human serum albumin) and, subsequently, freeze-drying it (Progress of Interferon Research, extra issue for "Protein, Nucleic Acid and Enzyme," No . 25, p. 358 (1981), Kyoritsu Shuppan), and so forth, are effective for stabilizing interferon. However, there is always a demand for a new method for stabilizing interferon. As the result of intensive studies of a method for stabilizing interferon, it has been found that the stability of interferon is remarkably enhanced by admixing the interferon with an inorganic salt such as sodium chloride, potassium chloride, etc., either alone or in combination with, for example, serum albumin.

Further, it has been found that the stability of interferon can also be remarkably increased by bringing interferon into contact with a saccharide alone or admixed with other additives.

Examples of interferon used in the present invention include natural interferon, interferon produced by incubation of animal cells, interferon produced by incubation of a microorganism obtained by recombinant DNA technology, etc., and also interferon of any of types $\alpha$, $\beta$ and $\gamma$ may be included. In the present invention, in particular, an excellent effect may be expected with Interferon-$\beta$ (hereinafter referred to as G-$\beta$-IFN) obtained by recombinant DNA technology.

As the non-toxic inorganic salt, alkali metal salt, such as sodium chloride, potassium chloride and lithium chloride, or magnesium chloride is used, and in particular, sodium chloride and potassium chloride are remarkable in the stabilizing effect.

In order to stabilize interferon, the inorganic salt is formulated; i.e., admixed with the interferon, in an amount of $1 \times 10^{-11}$ - $3 \times 10^{-10}$ mole per unit of interferon (international unit; "Latest Interferon" compiled by Kotaro Kishida, Asahi Science 1981), preferably, $3 \times 10^{-11}$ - $5 \times 10^{-11}$ mole per unit of interferon.

The inorganic salt exhibits a remarkable effect even when used alone and the effect may be even further enhanced by using it in combination with, for example, serum albumin, surfactants, antibiotics, chelating agents, amino acids, etc.

The conventional G-β-IFN freeze dried preparation employing serum albumin as a stabilizer becomes insoluble in water in 7 days or 14 days at 70°C, and its biological activity is reduced to 50 - 7%; whereas a G-β-IFN preparation containing serum albumin and sodium chloride can be readily dissolved in water in the same period of time, and about 90% of its biological activity remains.

Furthermore, the stability of G-β-IFN in an aqueous solution is examined by measuring the biological activity of $3 \times 10^6$ units/ml G-β-IFN under a temperature condition of 40°C. In the case where serum albumin is used, the G-β-IFN activity is reduced to 20% after 6 hours. On the other hand, where serum albumin and sodium chloride are used, 40% the G-β-IFN activity still remains after 6 hours.

As mentioned above, the stabilizing effect is achieved by bringing interferon into contact with a saccharide. Examples of a useful saccharide include dextran; e.g., dextran 40 (average molecular weight 40,000), and dextran 70 (average molecular weight 70,000), chondroitin sulfuric acid, starch, glycogen, inulin, dextrin, alginic acid salts, etc.

In order to stabilize interferon, the saccharide is formulated with the interferon in an amount of $5 \times 10^{-14}$ - $4 \times 10^{-12}$ mole per unit of interferon (international unit: a value determined based on an international standard sample; "Latest Interferon" in Asahi Science 1981), preferably, $1 \times 10^{-13}$ - $2 \times 10^{-12}$ mole per unit of interferon.

The saccharide exhibits an effect even when used alone, and the effect may be even further enhanced by using it in combination with inorganic salts, surfactants, serum albumin, antibiotics, chelating agents, amino acids, buffers, and the like additives which are non-toxic and pharmaceutically acceptable additives.

For example, when an inorganic salt (e.g., sodium chloride, potassium chloride, magnesium chloride, calcium chloride, etc.) is to be used in combination with the saccharide, the amount of the inorganic salt added is $1 \times 10^{-11} - 3 \times 10^{-10}$ mole per unit of interferon, and the amounts of the other additives may be, more or less, the same as that of the inorganic salt, although the amount to be added is dependent on their molecular weights, etc.

Further, the effect of a saccharide to stabilize interferon is more enhanced by storing a preparation containing interferon and a saccharide in a freeze-dried state rather than in a solution state.

The present invention also relates to compositions which contain interferon, and an inorganic salt or a saccharide. The amount of an inorganic salt or a saccharide to be contained in the compositions is the same as described for the above stabilizing method. Such compositions may incorporate, in addition to the inorganic salts or saccharides, pharmaceutically acceptable preservatives, stabilizers, binding agents, excipients, disintegrating agents, wetting agents, lubricants, coloring agents, aromatic agents, flavoring agents, coating agents, suspending agents, emulsifiers, dissolution aids, buffers, isotonic agents, plasticizers, plastic surfactants, etc.

Examples of the present invention are given below.

Example 1

(1) Preparation of G-β-IFN

Purified G-β-IFN was prepared according to the procedures described in European Patent Publication No. 42246, published on December 23, 1981 and about 200 μg of serum albumin (HSA: produced by CRYOSAN or BSA: produced by Seikagaku Kogyo) was added to about 5 μg of purified G-β-IFN.

(2) Preparation of Samples

Serum albumin and, as a stabilizer, an inorganic salt such as sodium chloride were added to the aforesaid G-β-IFN, and the mixture was freeze-dried. The preparation of G-β-IFN containing 10 mg of serum albumin and 5 mg of sodium chloride was used based on $3 \times 10^6$ units of G-β-IFN, while, as a control, that obtained by adding serum albumin alone was used.

One ml of distilled water were added to each of the freeze-dried products to prepare samples of aqueous solution.

(3)  Measurement of Interferon Activity

The potency of G-β-IFN was measured to determine the relative potency by simultaneously measuring standard G-β-IFN using a cytophatogenic inhibition - dye inclusion method by VSV virus employing WISH cells or FL cells (Progress of Interferon Research, extra issue for "Protein, Nucleic Acid and Enzyme," No. 25, p. 355 - 363 (1981), Kyoritsu Shuppan).

(4)  Stability Tests

(a)  Freeze-dried preparations

A storage stability test was conducted with the preparations having the compositions set forth in Table 1 in 7 days and 14 days in a constant temperature cell at 70°C.

The results are shown in Table 1.

Table  1

| No. | Serum Albumin (mg) | | Inorganic Salt (mg) | Remaining Activity of G-β-IFN (%) After | |
|-----|-----|-----|-----|-----|-----|
| | HSA | BSA | | 7 Days | 14 Days |
| 1 | 5 | - | - | 14 | 1 |
| 2 | 10 | - | - | 52 | 3 |
| 3 | - | 10 | - | 49 | 2 |
| 4 | 30 | - | - | 80 | 4 |
| 5 | 10 | - | 5 (NaCl) | 86 | 89 |
| 6 | 10 | - | 5 (KCl) | 82 | 79 |
| 7 | 10 | - | 5 ($MgCl_2$) | 75 | 72 |

(b)   Aqueous solutions

The freeze-dried preparations having the compositions set forth in Table 1 were dissolved in 1 ml of distilled water, put in a constant temperature cell at 40°C, and the potency in 6 hours and 24 hours were measured. The results are shown in Table 2.

Table   2

| No. | Remaining Activity of G-β-IFN (%) After | |
|---|---|---|
| | 6 Hours | 24 Hours |
| 1 | 18 | 16 |
| 2 | 23 | 15 |
| 3 | 22 | 14 |
| 4 | 44 | 17 |
| 5 | 45 | 20 |
| 6 | 43 | 19 |
| 7 | 40 | 18 |

Example 2

The storage stability of the G-β-IFN freeze-dried preparations was examined in the same manner as in Example 1 except that the amounts of the inorganic salts were changed to those given in Table 3.

The results are shown in Table 3.

Table 3

| No. | Inorganic Salt (mg) | | Remaining Activity of G-β-IFN (%) After | |
|---|---|---|---|---|
| | | | 7 Days | 14 Days |
| 2 | None | | 52 | 3 |
| 8 | 1 | (NaCl) | 60 | 31 |
| 9 | 3.5 | (NaCl) | 78 | 57 |
| 10 | 10 | (NaCl) | 81 | 55 |
| 11 | 11 | (KCl) | 86 | 83 |
| 12 | 9 | $(MgCl_2)$ | 79 | 76 |

Example 3

The storage stability of the freeze-dried preparations at 70°C was measured in the same manner as in Example 1 by using $6 \times 10^6$ units of purified G-β-IFN containing D-mannitol and inorganic salts as stabilizers.

The results are shown in Table 4.

Table 4

| No. | D-Mannitol (mg) | Inorganic Salt (mg) | | Remaining Activity (%) After | |
|---|---|---|---|---|---|
| | | | | 7 Days | 14 Days |
| 13 | 50 | None | | 7 | 0 |
| 14 | 50 | 10 | (NaCl) | 26 | 19 |
| 15 | 50 | 10 | (KCl) | 27 | 18 |

Example 4

The storage stability in an aqueous solution at 40°C was measured by using $1.8 \times 10^7$ units of purified G-β-IFN containing 30 mg of NaCl or KCl as a stabilizer.

- 8 -

0123291

The results are shown in Table 5.

Table 5

| No. | Inorganic Salt (mg) | Remaining Activity (%) After | |
|---|---|---|---|
| | | 1 Hour | 3 Hours |
| 16 | None | 11 | 2 |
| 17 | 30 (NaCl) | 30 | 14 |
| 18 | 30 (KCl) | 33 | 12 |

## Example 5

(1)  Preparation of G-β-IFN

G-β-IFN was prepared according to the procedures described in European Patent Publication No. 42246, published on December 23, 1981 and made into a 60% ethylene glycol - 2 M NaCl aqueous solution, which was used as a G-β-IFN ($2 \times 10^8$ U/ml).

(2)  Preparation of Samples

A saccharide, an inorganic salt and a buffer were added to the aforesaid G-β-IFN solution and the mixture was freeze-dried.  The preparation of G-β-IFN containing each of 10, 50 and 100 mg of dextran 40, 6 mg of NaCl and 2 ml of a 1/20 M $Na_2HPO_4$ - citric acid buffer (pH 4) based on $3 \times 10^6$ units of G-β-IFN was used while, as a control, a preparation containing the NaCl, the buffer and 50 mg of mannitol instead of dextran 40 was used.

(3)  Measurement of Interferon Activity

The potency of G-β-IFN was measured according to the same procedure as Example 1 (3).

(4)   Change in Potency by Freeze-Drying

(a)   $3 \times 10^6$ units of the G-β-IFN was added to each of solutions containing the respective additives set forth in Table 6, 6 mg of NaCl and 2 ml of a 1/20 M $Na_2HPO_4$ - citric acid buffer (pH 4), and the mixture was subjected to freeze-drying.   Freeze-dried samples were dissolved in water and diluted in Eagle's minimal essential medium supplemented with 5% fetal bovine serum.   The percent of the biological activity remaining after the freeze-drying relative to the biological activity before the freeze-drying was determined. The results are shown in Table 6.

Table   6

| No. | Saccharide | Amount (mg) | Percent of Activity of G-β-IFN Remaining Immediately After Freeze-Drying |
|-----|------------|-------------|--------------------------------------------------------------------------|
| 19 | Control (Mannitol) | 50 | 6 |
| 20 | Dextran 40 | 10 | 74 |
| 21 | Dextran 40 | 50 | 95 |
| 22 | Dextran 40 | 100 | 98 |

(b)   Upon changing the additives, as set forth in Table 7, the change in biological activity before and after the freeze-drying was again determined.

The results are shown in Table 7.

Table 7

| No. | Saccharide | Amount (mg) | Percent of Activity of G-β-IFN Remaining Immediately After Freeze-Drying |
|---|---|---|---|
| 19 | Control (Mannitol) | 50 | 6 |
| 21 | Dextran 40 | 50 | 95 |
| 23 | Dextran 70 | 10 | 82 |
| 24 | Dextran 70 | 50 | 97 |
| 25 | Chondroitin Sulfuric Acid | 50 | 72 |

Example 6

Freeze-dried samples were produced in the same manner as in Example 5 after changing the amount of sodium chloride used as the inorganic salt, and the storage stability at 70°C was studied.

The results are shown in Table 8.

Table 8

| No. | Saccharide | Amount (mg) | Sodium Chloride added (mg) | Remaining Activity of G-β-IFN (%) After | |
|---|---|---|---|---|---|
| | | | | 7 Days | 14 Days |
| 21 | Dextran 40 | 50 | 0 | 53 | 12 |
| 26 | Dextran 40 | 50 | 6 | 85 | 76 |
| 24 | Dextran 70 | 50 | 0 | 62 | 14 |
| 27 | Dextran 70 | 50 | 6 | 88 | 79 |

Example 7

The stability of an aqueous solution of $1.5 \times 10^6$ U/ml G-β-IFN prepared in Example 5 (1) was studied both in a buffer and in distilled water.

The results are shown in Table 9.

Table 9

| No. | Saccharide | Amount (mg) | Buffer (2 ml) | Percent of Activity of G-β-IFN Remaining After 6 Hrs. at 37°C |
|-----|-----------|-------------|---------------|---------------------------------------------------------------|
| 28 | Dextran 40 | 50 | Distilled water | 55 |
| 29 | Dextran 40 | 50 | 1/20 M N$_2$HPO$_4$ – Citric Acid Buffer (pH 4) | 88 |
| 30 | Dextran 70 | 50 | Distilled water | 62 |
| 31 | Dextran 70 | 50 | 1/20 M Na$_2$HPO$_4$ – Citric Acid Buffer (pH 4) | 93 |

1. A method for stabilizing interferon which comprises bringing an interferon into contact with a stabilizing agent containing an alkali metal salt, magnesium chloride or a saccharide selected from the group consisting of dextran, chondroitin sulfuric acid, starch, glycogen, inulin, dextrin and alginic acid salt.

2. The method according to claim 2, wherein the stabilizing agent is a serum albumin and an alkali metal salt.

3. The method according to claim 1 wherein the stabilizing agent is a saccharide defined in claim 1 and an alkali metal salt.

4. The method according to claim 1, 2 or 3, wherein the alkali metal salt is selected from the group consisting of sodium chloride, potassium chloride and lithium chloride.

5. The method according to claim 1, wherein the interferon is selected from the group consisting of Interferon- , Interferon- and Interferon- .

6. The method according to claim 1, wherein the amount of the alkali metal salt admixed with the interferon is $1 \times 10^{-11} - 3 \times 10^{-10}$ mole per unit of interferon.

7. The method according to claim 1, wherein the amount of saccharide admixed with the interferon is $5 \times 10^{-14} - 4 \times 10^{-12}$ mole per unit of interferon.

8. A composition which contains an interferon and a stabilizing agent containing alkali metal salt, magnesium chloride or saccharide selected from the group consisting of dextran, chondroitin sulfuric acid, starch, glycogen, inulin, dextrin and alginic acid salt.

9. The composition according to claim 8, wherein the stabilizing agent is a serum albumin and an alkali metal salt.

10. The composition according to claim 8, wherein the stabilizing agent is a saccharide defined in claim 8 and an alkali metal salt.

11. The composition according to claim 8, 9 or 10, wherein the alkali metal salt is selected from the group consisting of sodium chloride, potassium chloride and lithium chloride.

12. The composition according to claim 8, wherein the interferon is selected from the group consisting of Interferon- , Interferon-  and Interferon- .

13. The composition according to claim 8, wherein the amount of the alkali metal salt admixed with the interferon is $1 \times 10^{-11}$ - $3 \times 10^{-10}$ mole per unit of interferon.

14. The composition according to claim 8, wherein the amount of saccharide admixed with the interferon is $5 \times 10^{-14}$ - $4 \times 10^{-12}$ mole per unit of interferon.